# EUROPEAN PATENT SPECIFICATION

(11) **EP 1 466 938 B1**
(45) Date of publication and mention of the grant of the patent: **26.06.2019**
(21) Application number: 02790894.6
(22) Date of filing: 26.12.2002
(51) Int. Cl.: C08J 5/18, C08L 29/04

(54) **POLY(VINYL ALCOHOL) BASED FILM**
FOLIE AUF BASIS VON POLY(VINYLALKOHOL)
FILM A BASE DE POLY(ALCOOL DE VINYLE)

(30) Priority: 27.12.2001 JP 2001395801
(43) Date of publication of application: 13.10.2004
(73) Proprietor: The Nippon Synthetic Chemical Industry Co., Ltd., Osaka 530-0018 (JP)
(72) Inventor: KITAMURA, Shuichi, Ibaraki-shi 567-0052 Osaka (JP); MIZUTANI, Tomoyoshi, Ibaraki-shi 567-0052 Osaka (JP)
(74) Representative: TBK
(86) International application number: PCT/JP2002/013657
(87) International publication number: WO 2003/055938

(56) References cited:
- EP-A- 1 158 016
- JP-A- 2 151 639
- JP-A- 8 041 427
- JP-A- 9 302 161
- JP-A- 9 324 096
- JP-A- 9 324 096
- JP-A- 59 152 852
- JP-A- 2001 192 011
- JP-A- 2002 275 339

## Description

### TECHNICAL FIELD

The present invention relates to a polyvinyl alcohol film (polyvinyl alcohol being hereinafter referred to as PVA) having an excellent solubility in cold water, and more particularly to a water-soluble PVA film having an excellent durability such that change in appearance of the film under a high humidity is slight and, even if the film is stored for a long term, the cold water solubility is only slightly decreased.

### BACKGROUND ART

PVA films have been used, utilizing the water solubility, as a material for unit-dose packaging (unit pack) of chemicals such as agricultural chemicals and detergents, a film for (water pressure) transfer printing, sanitary goods such as sanitary napkin and paper diaper, filth-treating goods such as ostomy bag, medical supplies such as blood-adsorbing sheet, temporary substrates such as sheet for seedling culture, seed tape or foundation for embroidery, and the like In particular, unit-dose packages of chemicals such as agricultural chemicals and detergents have the advantages that time for weighing for each occasion of use can be saved and there is no occurrence of getting hands dirty.

As a PVA of water-soluble films used for these purposes has been generally used a PVA with a degree of hydrolysis of about 80 to about 90 % by mole which has a water solubility, particularly a water solubility at low temperatures (cold water solubility). However, films of PVA with such a low degree of hydrolysis are still insufficient in solubility at low temperatures. In particular, in case of packaging alkaline substances with the films in unit packaging, problems arise that the degree of hydrolysis progresses by contact with the alkaline substances to result in decrease of cold water solubility, and change in appearance of the films such as wrinkling or elongation is caused by influence of humidity.

In order to improve the cold water solubility, for instance, JP-A-43-1487 proposes a PVA film comprising a PVA having a high degree of hydrolysis of at least 97 % by mole, a PVA having a low degree of hydrolysis within the range of 75 to 92 % by mole and starch. JP-A-63-168437 proposes a PVA film for packaging alkaline substances, prepared by forming a PVA containing at least one of oxyalkylene group), sulfo group and cationic group into a film. JP-A-10-060207 proposes a water-soluble film comprising a modified PVA resin having an anionic group modification ratio of 2.0 to 40.0 % by mole and a water-insoluble or slightly water-soluble fine powder having an average particle size of at most 150 µm. These water-soluble films have an improved cold water solubility, but the cold water solubility is still unsatisfactory for uses requiring quick solubility. Further, these water-soluble films still have a problem in durability that change in appearance of film caused by wrinkling or elongation occurs when the films are allowed to stand under a high humidity. Thus, further improvement has been demanded.

EP1158016 (A2) provides a film for packaging chemical agents, which contains an oxyalkylene unit-containing polyvinyl alcohol resin (A) and a carboxyl-containing polyvinyl alcohol resin (B), and preferably further containing a sulfo-containing polyvinyl alcohol resin (C).

JP-H09-324096 (A) relates to a composition comprising a modified PVA resin having anionic groups (pref. carboxyl and/or sulfo groups) preferably in an amount of 2.0-40mol% (in terms of degree of modification) and a degree of polymerization of preferably 200-8,000 and a PVA resin having a degree of saponification of 70-99mol% and a degree of polymerization of preferably 200-8,000.

JP2001106854 (A) relates to water-soluble film composed of a resin composition that is obtained by compounding 1-50 pts.wt. plasticizer and 5-50 pts.wt. starch with 100 pts.wt. sulfonic acid modifiec polyvinyl alcohol having a saponification degree of not less than 90 mol%.

Accordingly, under such circumstances, it is an object of
the present invention to provide a PVA film having an excellent cold water solubility and having an excellent durability such that change in appearance of the film under high humidity is slight and the cold water solubility of the film is only slightly decreased even if unit-dose packages made therefrom are stored for a long term.

### DISCLOSURE OF INVENTION

As a result of making intensive study in order to solve such problems, the present inventors have found that a polyvinyl alcohol film having a solubility that it can dissolve in water at 20°C in 10 minutes, wherein the α/β ratio of the storage modulus α of the film at 20°C in a dry atmosphere to the storage modulus β of the film at 20°C and 80 %RH is not more than 10, meets the above object.

Thus, in accordance with the present invention, there is provided a polyvinyl alcohol film as defined by claim 1.

Preferably, the film of the present invention is prepared from a resin composition [I] containing at least two kinds of PVA resins (A) having different degrees of hydrolysis. Resin composition [I] further containing an inorganic filler (B) is also preferable.

The phrase "soluble in water at 20°C within 10 minutes" as used herein denotes that when a specimen having a size of 5 cm × 5 cm is cut from a film, fixed to a tool and immersed in water (1 liter) kept at 20°C in a 1 liter beaker with stirring with a stirrer, the time up to the dissolution of the specimen is within 10 minutes. The term "dissolution" means that the specimen can no longer be visually observed, but herein encompasses the state that insoluble fine particles having a diameter of not more than 1 mm are dispersed in water.

Also, the term "dry atmosphere" means the state that the moisture content is not more than 1,000 ppm. Further, the "storage modulus" denotes a value measured when a vibration of a specific frequency is applied to a film. In the present invention, using a humidity conditioning visco-elastometer, (1) the storage modulus of a film is continuously measured under conditions of 20°C and dry atmosphere at a measuring frequency of 2 Hz with raising the temperature of the film from -50 to 150°C at a rate of 3°C/minute, and the measured value at 20°C is taken as the storage modulus a, and (2) the storage modulus of a film is continuously measured under conditions of 20°C and 80 %RH at a measuring frequency of 2 Hz with raising the temperature of the film from 10 to 50°C at a rate of 3°C/minute, and the measured value at 20°C is taken as the storage modulus β.

### BEST MODE FOR CARRYING OUT THE INVENTION

The PVA film of the present invention is required to have a solubility that it dissolves in water at 20°C within 10 minutes. It is also required for the PVA film of the invention that the α/β ratio of the storage modulus α of the film in a dry atmosphere at 20°C to the storage modulus β of the film at 20°C and 80 %RH is not more than 10. The storage modulus α/β ratio is preferably not more than 8, and a preferable lower limit of the α/β ratio is 3 or more. If the cold water solubility of the film is more than 10 minutes, there occurs inconvenience such that when washing is conducted using a detergent packed in the film, the detergent is not well released into water or a part of the film adheres to clothes, or when unit-dose packages are stored for a long term, the film becomes insoluble in water. If the storage modulus α/β ratio is more than 10, there arise problems that when unit-dose packages are stored for a long term, the packaging film is wrinkled, causes blocking or decreases its water solubility.

It is preferable that the storage modulus α is from 10⁶ to 10⁸ Pa. On the other hand, the storage modulus β may be any value so long as the α/β ratio is not more than 10 wherein the storage modulus α is from 10⁶ to 10⁸ Pa. If the storage modulus α is less than 10⁶ Pa, the film is short of strength required when packaging, and causes inconvenience such that the film cannot withstand an apparatus such as automatic filling machine used for filling contents or cannot withstand the weight of the contents. If the storage modulus α is more than 10⁸ Pa, the contents tend to leak outside the packages since pinholes may be formed when processing the film into bags or the packages are cracked owing to impact when packaging the contents and transporting the packages.

In the present invention, a process for preparing the PVA film satisfying the above requirements is not particularly limited, and there are mentioned, for instance, (1) a process wherein a resin composition containing at least two kinds of PVA resins having different degrees of hydrolysis is formed into a film, (2) a process wherein a resin composition containing a PVA resin soluble in cold water of 20°C and 1 to 50 parts by weight of an inorganic filler per 100 parts by weight of the PVA resin is formed into a film, and (3) a process wherein a PVA resin and a water-soluble resin other than the PVA resin are blended and formed into a film. Of these, the process (1) is preferable from the viewpoint of cost. The process (1) may be used in combination with process (2) and/or process (3). An explanation is given below particularly with respect to process (1).

PVA resin (A) used in the present invention can be prepared by a know method and is
obtained by polymerizing a vinyl ester compound and hydrolyzing the resulting vinyl ester polymer.

The vinyl ester compound is selected from vinyl formate, vinyl acetate, vinyl trifluroacetate, vinyl propionate, vinyl butyrate, vinyl caprate, vinyl laurate, vinyl ester of Versatics, vinyl palmitate, and vinyl stearate. These may be used alone or in
admixture thereof. Vinyl acetate is suitable from a practical point of view.

The polymerization can be conducted by any of known polymerization methods without particular restriction. Usually, a solution polymerization using an alcohol such as methanol, ethanol or isopropyl alcohol as a solvent is practiced. Emulsion polymerization and suspension polymerization are of course possible. The polymerization reaction is conducted using a known radical polymerization initiator such as azobisisobutylonitrile, acetyl peroxide, benzoyl peroxide or lauroyl peroxide. The reaction temperature is selected from the range between about 35°C and the boiling point of a solvent, especially 40 to 80°C, more especially 50 to 80°C.

The hydrolysis of the obtained vinyl ester polymer is conducted according to a known method by dissolving the polymer in an alcohol and hydrolyzing it in the presence of an alkali catalyst. Examples of the alcohol are methanol, ethanol, butanol and the like. The concentration of the polymer in the alcohol is selected within the range of 20 to 50 % by weight.

As the hydrolysis catalyst are used alkali catalysts, e.g., hydroxide and alcoholate of an alkali metal such as sodium hydroxide, potassium hydroxide, sodium methylate, sodium ethylate or potassium methylate. The amount of such a catalyst is from 1 to 100 millimolar equivalents based on the vinyl ester compound. If circumstances require, it is possible to conduct the hydrolysis by using an acid catalyst.

In the present invention, it is preferable to prepare a film from a resin composition [I] containing at least two kinds of PVA resins (A) having different degrees of hydrolysis. The degree of hydrolysis is selected from the range of 55 to 100 % by mole, preferably 60 to 100 % by mole, more preferably 70 to 100 % by mole.

In the case where at least two kinds of PVA resins (A) having different degrees of hydrolysis are used, it is preferable that the difference in degree of hydrolysis between the respective PVA resins is at least 3 % by mole, especially 3 to 20 % by mole, more especially 5 to 18 % by mole. In particular, it is preferable that the resin composition [I] comprises two kinds of PVA resins (A) having different degrees of hydrolysis, and the difference in degree of hydrolysis between a PVA resin (a1) having a lower degree of hydrolysis and a PVA resin (a2) having a higher degree of hydrolysis is at least 3 % by mole, especially 3 to 20 % by mole, more especially 5 to 18 % by mole. If the difference in degree of hydrolysis between PVA resin (a1) and PVA resin (a2) is less than 3 % mole, it is difficult to achieve both the cold water solubility of the film and the effect of suppressing occurrence of wrinkling and elongation under a high humidity.

Further, in the present invention, it is preferable that the degree of hydrolysis of the PVA resin (a1) having a lower degree of hydrolysis is at least 55 % by mole, preferably at least 60 % by mole, more preferably not less than 70 % by mole and less than 82 % by mole, especially not more than 80 % by mole. On the other hand, it is preferable that the degree of hydrolysis of the PVA resin (a2) having a higher degree of hydrolysis is at least 82 % by mole, preferably at least 88 % by mole, and is preferably not more than 99.99 % by mole. If the degree of hydrolysis of the PVA resin (a1) is less than 55 % by mole, the solvent resistance of the obtained film is deteriorated, and if it is not less than 82 % by mole, the cold water solubility is deteriorated. If the degree of hydrolysis of the PVA resin (a2) is less than 82 % by mole, the strength of the film is drastically deteriorated under a high humidity.

The viscosity of a 4 % by weight aqueous solution of each of PVA resins (a1) and (a2) measured at 20°C is not particularly limited, but preferably the viscosity for PVA resin (a1) is from 2 to 70 mPa·s, especially from 2 to 60 mPa·s,and preferably the viscosity for PVA resin (a2) is also from 2 to 70 mPa·s, especially 2 to 60 mPa·s. If the viscosity of PVA resin (a1) is less than 2 mPa·s, the mechanical strength of films is not enough, and if it is more than 70 mPa·s, the viscosity of an aqueous solution prepared for film formation becomes high, so the productivity is lowered. On the other hand, the same is true for PVA resin (a2). If the viscosity is less than 2 mPa·s, the mechanical strength of films is not enough, and if it is more than 70 mPa·s, the viscosity of an aqueous solution prepared for film formation becomes high, so the productivity is lowered.

The proportions of PVA resin (a1) and PVA resin (a2) are not particularly limited. Preferably the a1/a2 ratio is from 50/50 to 90/10 by weight, especially from 55/45 to 80/20 by weight. If the a1/a2 ratio is less than 50/50 by weight, the cold water solubility is deteriorated, and if it is more than 90/10 by weight, wrinkling and elongation are easy to occur under a high humidity.

The PVA film of the present invention is obtained by forming a resin composition [I] comprising PVA resins (a1) and (a2) into film. In the present invention, it is preferable that the resin composition [I] further contains an inorganic filler (B) as well as at least two kinds of PVA resins (A) having different degrees of hydrolysis. Incorporation of an inorganic filler having an average particle size of not more than 10 µm improves the cold water solubility of the obtained PVA films.

It is preferable that the inorganic filler (B) is a powder having an average particle size of 1 to 10 µm. If the average particle size is less than 1 µm, the cold water solubility of films is not improved, and the effect of preventing blocking of films is small. If it is more than 10 µm, the appearance of films is deteriorated to decrease the commodity value.

Conventional inorganic fillers and other inorganic powder can be used as the inorganic filler (B) without particular restriction. Examples of the inorganic filler are, for instance, talc, clay, silicon dioxide, diatomaceous earth, kaolin, mica, asbestos, gypsum, graphite, glass balloon, glass beads, calcium sulfate, barium sulfate, ammonium sulfate, calcium sulfite, calcium carbonate, whisker-like calcium carbonate, magnesium carbonate, dawsonite, dolomite, potassium titanate, carbon black, glass fiber, alumina fiber, boron fiber, processed mineral fiber, carbon fiber, carbon hollow beads, bentonite, montmorillonite, copper powder, sodium sulfate, potassium sulfate, zinc sulfate, copper sulfate, iron sulfate, magnesium sulfate, aluminum sulfate, potassium aluminum sulfate, ammonium nitrate, sodium nitrate, potassium nitrate, aluminum nitrate, ammonium chloride, sodium chloride, potassium chloride, magnesium chloride, calcium chloride, sodium phosphate, potassium chromate, calcium citrate, and the like.

The amount of the inorganic filler (B) is not particularly limited, but is preferably from 0.1 to 50 parts by weight, more preferably from 0.5 to 10 parts by weight, per 100 parts by weight of the total of PVA resins (A). If the amount of the filler (B) is less than 0.1 part by weight, the cold water solubility is not improved, and the effect of preventing blocking of films is small. If the amount is more than 50 parts by weight, the tensile elongation of films is deteriorated.

Further, in the present invention, for the purpose of improving the flexibility of films at low temperatures and the workability in the preparation of films,
a plasticizer (C) is incorporated into the PVA film.

Plasticizer conventionally used in PVA films can be used herein. Examples of the plasticizer (C) are, for instance, glycerol, diglycerol, polyethylene glycol, polypropylene glycol, trimethylolpropane, reducing maltose (reducing starch hydrolyzate by malt), reducing lactose, reducing starch sugar, sorbitol, mannitol, xylitol, and the like. The olasticizers may be used alone or in admixture thereof. At least trimethylolpropane is used.

The amount of the plasticizer (C) is not particularly limited, but is preferably from 0.1 to 50 parts by weight, more preferably from 1 to 40 parts by weight, per 100 parts by weight of the total of the PVA resins (A). If the amount is less than 0.1 part by weight, the plasticizing effect is low. If the amount is more than 50 parts by weight, the plasticizer is easy to bleed to the surface of film with the lapse of time.

The resin composition [I] may be further incorporated with a starch having an average particle size of more than 10 µm, especially not less than 15 µm, for the purpose of preventing blocking or adjusting mechanical strength. Examples of the starch are, for instance, a raw starch such as corn starch, potato starch, sweet potato starch, wheat starch, cassava starch, sago starch, tapioka starch, corn starch, rice starch, bean starch, kudzu starch, bracken starch, lotus starch or water chestnut starch; a physically modified starch such as α-starch, discrete amylose or moist heat processed starch; an enzyme-modified starch such as hydrolyzed dextrin, enzyme decomposed dextrin or amylose; a chemically decomposed starch such as acid treated starch, hypochlorous acid-oxidized starch or dialdehyde starch; a chemically modified starch derivative such as esterified starch, etherified starch, cationized starch or crosslinked starch, and the like. The esterified starch includes acetic acid esterified starch, succinic acid esterfied starch, nitric acid esterified starch, phosphoric acid esterified starch, urea-phosphoric acid esterified starch, xanthic acid esterified starch, acetoacetic acid esterified starch, and the like. The etherified starch includes allyl etherified starch, methyl etherified starch, carboxymethyl etherified starch, hydroxyethyl etherified starch, hydroxypropyl etherified starch, and the like. The cationized starch includes a reaction product of starch and 2-diethylaminoethyl chloride, a reaction product of starch and 2,3-epoxypropyltrimethylammonium chloride, and the like. The crosslinked starch includes formaldehyde-crosslinked starch, epichlorohydrin-crosslinked starch, phosphoric acid-crosslinked starch, acrolein-crosslinked starch, and the like. Of these, raw starches are preferable from the viewpoints of availability and economy.

The amount of such a starch is not particularly limited, but is preferably from 0.1 to 40 parts by weight, more preferably from 1 to 30 parts by weight, per 100 parts by weight of the total of the PVA resins (A). If the amount is less than 0.1 part by weight, anti-blocking effect and mechanical strength improving effect are small. If the amount is more than 40 parts by weight, the appearance and elongation of films are markedly lowered.

Thus, in the present invention, PVA films are obtained by forming films from resin composition [I] containing two or more PVA resins (A) having different degrees of hydrolysis, preferably two PVA resins (a1) and (a2) having different degrees of hydrolysis from each other by at least 3 % by mole, preferably the resin composition [I] which further contains inorganic filler (B) and/or plasticizer (C). The film formation can be carried out by known methods, e.g., casting, without particular restriction.

The film formation by casting will be explained below. Water is added to the above-mentioned resin composition [I] which is in the form of powder, to give an aqueous solution of the resin composition [I] having a solid concentration of 10 to 50 % by weight, preferably 15 to 35 % by weight. Alternatively, water is added to a powder of PVA resin (A) to give an aqueous solution of the PVA resin (A) having a solid concentration of 10 to 50 % by weight, preferably 15 to 35 % by weight, and additives such as inorganic filler (B) and plasticizer (C) are further added to the obtained aqueous solution to give an aqueous dispersion of resin composition [I] having a solid concentration of 10 to 50 % by weight, preferably 15 to 35 % by weight.

To the thus prepared aqueous dispersion or solution may be further added, as occasion demands, a surfactant in an amount of 0.1 to 10 parts by weight, preferably 0.3 to 5 parts by weight, per 100 parts by weight of the total of the PVA resins (A). Examples of the surfactant are, for instance, polyoxyethylene nonyl phenyl ether, polyoxyethylene octyl nonyl ether, polyoxyethylene dodecyl phenyl ether, a polyoxyethylene alkyl allyl ether, polyoxyethylene sorbitan monolaurate, polyoxyethylene sorbitan monopalmitate, polyoxyethylene sorbitan monostearate, polyoxyethylene sorbitan monooleate, a polyoxyalkylene alkyl ether phosphate monoethanolamine salt, a polyoxyethylene alkylamine such as polyoxyethylene laurylamine or polyoxyethylene stearylamine, and the like.

Such an aqueous solution or dispersion is then passed through a slit such as T-die, cast onto the surface of a metal roll or metal drum having a surface temperature of about 90 to about 100°C, dried and optionally heated-treated, thus providing the PVA films of the present invention. The PVA films can also be prepared by casting the aqueous solution or dispersion of the resin composition [I], using an applicator, onto a plastic substrate such as polyethylene terephthalate film or polyethylene film or a metal substrate, and drying it. The preparation of PVA films has been explained with respect to casting method, but is not limited thereto in the present invention.

The PVA films of the present invention have a glass transition temperature of -10 to 15°C, more especially -5 to 10°C. If the glass transition temperature of the films is more than 20°C, change in mechanical strength of the films depending on environment becomes large. The glass transition temperature of not higher than 20°C can be achieved by suitably adjusting the kind and amount of the plasticizer, the degree of hydrolysis of the PVA resins, the heat treating temperature in the film formation, or the content of water in the films.

The term "glass transition temperature" of the PVA films as used herein means the peak temperature of main dispersion obtained when continuously measuring the dynamic viscoelasticity in a dry atmosphere of 20°C at a measuring frequency of 2 Hz with elevating the temperature from -50°C to 150°C at a rate of 3°C/minute by using a humidity conditioning visco-elastometer (model DVA-225 made by IT Keisokuseigyo Kabushiki Kaisha). The "dry atmosphere" means, as stated before, the state that the moisture content is not more than 1,000 ppm.

The thickness of the PVA films of the present invention may vary depending on purposes, but in general it is preferable that the thickness is from 5 to 100 µm, especially 10 to 80 µm. If the thickness is less than 5 µm, the mechanical strength of the films is low, and if the thickness is more than 100 µm, the rate of dissolution of the films into cold water is very slow and the film formation efficiency is also low.

The surface of the PVA films may be plane, or either or both surfaces of the films may be provided with embossed pattern or satin crape finish.

The PVA films of the present invention may contain usual additives, so long as the objects of the present invention are not impaired, e.g., other water-soluble polymers (polyacrylic acid sodium salt, polyethylene oxide, polyvinyl pyrrolidone, dextrin, chitosan, chitin, methyl cellulose, hydroxyethyl cellulose, etc.), a rust inhibitor, a colorant, and the like. The ratio (A/D) of the PVA resin (A) to the water-soluble polymer (D) is usually from 80/20 to 20/80 by weight.

The thus obtained PVA films have an excellent cold water solubility. Further, the appearance change of the films under high humidity is slight, and the lowering of the cold water solubility is slight even if the films are stored for a long term. Therefore, the films are useful as water-soluble films for various purposes, and can be utilized for purposes, e.g., a material for packaging (unit-dose packaging) of chemicals such as agricultural chemicals and detergents, a film for (water pressure) transfer printing, sanitary goods such as sanitary napkin and paper diaper, filth-treating goods such as ostomy bag, medical supplies such as blood-adsorbing sheet, temporary substrates such as sheet for seedling culture or foundation for embroidery, and the like. In particular, the films are very useful for use in packaging of chemicals.

In case of using for the purpose of packaging of chemicals, the PVA films of the present invention can be suitably applied even to chemicals which are liquid at ordinary temperature (e.g., liquid detergents), to say nothing of powdery chemicals. In particular, the effects of the present invention are noticeably exhibited when the chemicals are liquid.

The present invention is more specifically described and explained by means of the following Examples in which all parts and % are by weight unless otherwise noted.

### EXAMPLE 1

An aqueous dispersion of resin composition [I] having a solid concentration of 15 % was prepared by mixing 60 parts of a PVA resin (a1) having a degree of hydrolysis of 72 % by mole and a 4 % aqueous solution viscosity of 6 mPa·s at 20°C, 40 parts of a PVA resin (a2) having a degree of hydrolysis of 98.5 % by mole and a 4 % aqueous solution viscosity of 5 mPa·s at 20°C, 2 parts of silicon dioxide (B) having an average particle size of 6.4 µm, 20 parts of trimethylolpropane (C), and 690 parts of water.

The obtained aqueous dispersion was cast onto a polyethylene terephthalate (PET) film by using an applicator and then dried at 90°C for 10 minutes to give a PVA film having a thickness of 60 µm.

The obtained PVA film was soluble in water of 20°C within 10 minutes. Further, the storage modulus α of the film in a dry atmosphere at 20°C was 4.0 × 10⁷ Pa, and the storage modulus β of the film at 20°C and 80 %RH was 6.0 × 10⁶ Pa, thus the α/β ratio was 6.7. Also, the obtained PVA film had a glass transition temperature of 6°C.

With respect to the obtained PVA film, the following evaluation was made. The results are shown in Table 1.

### Initial solubility in cold water

The obtained PVA film was cut to a size of 3 cm × 5 cm and fixed to a tool. One liter of water was placed in an 1 liter beaker, and the film was immersed in water with stirring by a stirrer and keeping the water temperature at 5°C. The stirring was continued and the time up to the dissolution of the film was measured. Herein the "dissolution" means that the film can no longer be visually observed, but encompasses the state that insoluble fine particles having a diameter of not more than 1 mm are dispersed in water.

### Durability

### (1) Change in appearance of film

The obtained PVA film was cut to a size of 6 cm × 9 cm, and two sheets of the cut film were sealed at the three sides by a heat sealer to form a bag. The bag was charged with 40 g of glycerol and hermetically sealed by a heat sealer. After allowing it to stand for two weeks in an atmosphere of 27°C and 80 %RH, the change (wrinkling and elongation) in appearance of the film was evaluated according to the following criteria.
○: Wrinkling and elongation are scarcely observed.
×: Wrinkling and elongation are clearly observed.

### (2) Solubility in cold water

A bag was prepared from the obtained PVA film in the same manner as above, and it was charged with 40 g of glycerol and sealed. After allowing the bag to stand for four weeks in an atmosphere of 27°C and 80 %RH, the bag was cut to obtain a film having a size of 3 cm × 5 cm. The time up to the dissolution of the film was measured in the same manner as in the above evaluation of initial solubility in cold water.

### EXAMPLE 2

A PVA film was prepared in the same manner as in Example 1 except that 70 parts of a PVA resin having a degree of hydrolysis of 72 % by mole and a 4 % aqueous solution viscosity of 6 mPa·s (at 20°C) was used as a PVA resin (a1), and 30 parts of a PVA resin having a degree of hydrolysis of 98.5 % by mole and a 4 % aqueous solution viscosity of 5 mPa·s (at 20°C) was used as a PVA resin (a2).

The obtained PVA film was soluble in water of 20°C within 10 minutes. Further, the storage modulus α of the film in a dry atmosphere at 20°C was 3.3 × 10⁷ Pa, and the storage modulus β of the film at 20°C and 80 %RH was 4.3 × 10⁶ Pa, thus the α/β ratio was 7.7 Also, the obtained PVA film had a glass transition temperature of 8°C.

The obtained PVA film was evaluated in the same manner as in Example 1. The results are shown in Table 1.

### EXAMPLE 3

A PVA film was prepared in the same manner as in Example 1 except that 70 parts of a PVA resin having a degree of hydrolysis of 72 % by mole and a 4 % aqueous solution viscosity of 6 mPa·s (at 20°C) was used as a PVA resin (a1), and 30 parts of a PVA resin having a degree of hydrolysis of 88 % by mole and a 4 % aqueous solution viscosity of 5 mPa·s (at 20°C) was used as a PVA resin (a2).

The obtained PVA film was soluble in water of 20°C within 10 minutes. Further, the storage modulus α of the film in a dry atmosphere at 20°C was 3.7 × 10⁷ Pa, and the storage modulus β of the film at 20°C and 80 %RH was 7.2 × 10⁶ Pa, thus the α/β ratio was 5.1. Also, the obtained PVA film had a glass transition temperature of 8°C.

The obtained PVA film was evaluated in the same manner as in Example 1. The results are shown in Table 1.

### COMPARATIVE EXAMPLE 1

A PVA film was prepared in the same manner as in Example 1 except that an aqueous PVA solution having a solid concentration of 15 % was prepared by mixing 100 parts of a PVA resin (a2) having a degree of hydrolysis of 98.5 % by mole and a 4 % aqueous solution viscosity of 5 mPa·s (at 20°C), 2 parts of silicon dioxide (B) having an average particle size of 6.4 µm, 10 parts of glycerol (C), and 635 parts of water.

The obtained PVA film was not soluble in water of 20°C within 10 minutes. Further, the storage modulus α of the film in a dry atmosphere at 20°C was 1.2 × 10⁹ Pa, and the storage modulus β of the film at 20°C and 80 %RH was 2.8 × 10⁷ Pa, thus the α/β ratio was 42. Also, the obtained PVA film had a glass transition temperature of 25°C.

The obtained PVA film was evaluated in the same manner as in Example 1. The results are shown in Table 1.

### COMPARATIVE EXAMPLE 2

A PVA film was prepared in the same manner as in Example 1 except that an aqueous PVA solution having a solid concentration of 15 % was prepared by mixing 100 parts of a PVA resin (a1) having a degree of hydrolysis of 72 % by mole and a 4 % aqueous solution viscosity of 6 mPa·s (at 20°C), 2 parts of silicon dioxide (B) having an average particle size of 6.4 µm, 10 parts of glycerol (C), and 635 parts of water.

The obtained PVA film was soluble in water of 20°C within 10 minutes. Further, the storage modulus α of the film in a dry atmosphere at 20°C was 2.8 × 10⁸ Pa, and the storage modulus β of the film at 20°C and 80 %RH was 4.4 × 10⁶ Pa, thus the α/β ratio was 64. Also, the obtained PVA film had a glass transition temperature of 30°C.

The obtained PVA film was evaluated in the same manner as in Example 1. The results are shown in Table 1.

### COMPARATIVE EXAMPLE 3

A PVA film was prepared in the same manner as in Example 1 except that 60 parts of a PVA resin having a degree of hydrolysis of 72 % by mole and a 4 % aqueous solution viscosity of 6 mPa·s (at 20°C) was used as a PVA resin (a1), and 40 parts of a PVA resin having a degree of hydrolysis of 88 % by mole and a 4 % aqueous solution viscosity of 5 mPa·s (at 20°C) was used as a PVA resin (a2)

The obtained PVA film was not soluble in water of 20°C within 10 minutes. Further, the storage modulus α of the film in a dry atmosphere at 20°C was 8.7 × 10⁷ Pa, and the storage modulus β of the film at 20°C and 80 %RH was 8.3 × 10⁶ Pa, thus the α/β ratio was 11. Also, the obtained PVA film had a glass transition temperature of 16°C.

The obtained PVA film was evaluated in the same manner as in Example 1. The results are shown in Table 1.

**Table 1**

| | Initial cold water solubility (second) | Durability | |
|---|---|---|---|
| | | Change in film appearance | Cold water solubility (second) |
| Ex. 1 | 50 | ○ | 82 |
| Ex. 2 | 50 | ○ | 82 |
| Ex. 3 | 60 | ○ | 88 |
| Com. Ex. 1 | insoluble | ○ | insoluble |
| Com. Ex. 2 | 50 | × | 85 |
| Com. Ex. 3 | 75 | ○ | 101 |

### INDUSTRIAL APPLICABILITY

Since the PVA film of the present invention is a film soluble in water at 20°C within 10 minutes and the α/β ratio of the storage modulus α of the film at 20°C in a dry atmosphere to the storage modulus β of the film at 20°C and 80 %RH is not more than 10, it has the effects such that the cold water solubility is excellent, the change in appearance of the film under high humidity is slight, and the lowering of the cold water solubility is slight even if the film is stored for a long term. The film is useful as a water-soluble film for various purposes, and is useful for purposes, e.g., a material for packaging (unit-dose packaging) of chemicals such as agricultural chemicals and detergents, a film for (water pressure) transfer printing, sanitary goods such as sanitary napkin and paper diaper, filth-treating goods such as ostomy bag, medical supplies such as blood-adsorbing sheet, and temporary substrates such as sheet for seedling culture or foundation for embroidery, particularly packaging of chemicals.

## Claims

1. A polyvinyl alcohol (PVA) film which dissolves in water at 20°C within 10 minutes if a specimen having a size of 5 cm x 5 cm is cut from a film having a thickness between 5 and 100 µm and immersed in 1 L of water kept at 20°C and stirred with a stirrer, and has an α/β ratio of not more than 10, wherein α is a storage modulus of the film at 20°C in a dry atmosphere and β is a storage modulus of the film at 20°C and 80 %RH, wherein the PVA is obtained by polymerizing a vinyl ester compound selected from vinyl formate, vinyl acetate, vinyl trifluoroacetate, vinyl propionate, vinyl butyrate, vinyl caprate, vinyl laurate, vinyl ester of Versatics, vinyl palmitate, and vinyl stearate, whereby the PVA film has a glass transition temperature, which is the peak temperature of main dispersion obtained when continuously measuring the dynamic viscoelasticity in a condition of 20°C with a moisture content of not more than 1,000 ppm, at a measuring frequency of 2 Hz with elevating the temperature from -50°C to 150°C at a rate of 3°C/minute by using a humidity conditioning visco-elastometer, in a range of -10°C to 15°C, and wherein the PVA film further contains a plasticizer (C) comprising trimethylolpropane.

2. The film of claim 1, which is prepared by forming a film from a resin composition [I] containing at least two kinds of polyvinyl alcohol resins (A) having different degrees of hydrolysis.

3. The film of claim 2, wherein said resin composition [I] comprises two kinds of polyvinyl alcohol resins (A) having different degrees of hydrolysis, and the difference in degree of hydrolysis between a polyvinyl alcohol resin (a1) having a lower degree of hydrolysis and a polyvinyl alcohol resin (a2) having a higher degree of hydrolysis is at least 3 % by mole.

4. The film of claim 3, wherein said polyvinyl alcohol resin (a1) has a degree of hydrolysis of not less than 70 % by mole to less than 82 % by mole, and said polyvinyl alcohol resin (a2) has a degree of hydrolysis of not less than 82 % by mole.

5. The film of claim 3 or 4, wherein the ratio of polyvinyl alcohol resin (a1) to polyvinyl alcohol resin (a2) is from 50/50 to 90/10 by weight.

6. The film of any one of claims 1 to 5, which further contains an inorganic filler (B) having an average particle size of 1 to 10 µm.

7. A package of a chemical comprising a polyvinyl alcohol film of any one of claims 1 to 6, and a chemical.

8. The package of claim 7, wherein said chemical charged is a liquid at ordinary temperature.

## Patentansprüche

1. Polyvinylalkohol-(PVA)-Folie, die sich in Wasser bei 20°C innerhalb von 10 Minuten auflöst, wenn eine Probe mit einer Größe von 5 cm x 5 cm aus einer Folie mit einer Dicke zwischen 5 und 100 µm geschnitten und in 1 L Wasser eingetaucht wird, das bei 20°C gehalten und mit einem Rührwerk gerührt wird, und die ein α/β-Verhältnis von nicht mehr als 10 aufweist, wobei α ein Speichermodul der Folie bei 20°C in einer trockenen Atmosphäre und β ein Speichermodul der Folie bei 20°C und 80 %RH ist, wobei der PVA durch Polymerisieren einer Vinylesterverbindung ausgewählt aus Vinylformiat, Vinylacetat, Vinyltrifluoracetat, Vinylpropionat, Vinylbutyrat, Vinylcaprat, Vinyllaurat, Vinylester von Versatics, Vinylpalmitat und Vinylstearat erhalten wird, wobei die PVA-Folie eine Glasübergangstemperatur, die die Peaktemperatur der Hauptdispersion ist, die bei der kontinuierlichen Messung der dynamischen Viskoelastizität in einem Zustand von 20°C mit einem Feuchtigkeitsgehalt von nicht mehr als 1000 ppm, bei einer Messfrequenz von 2 Hz, mit Erhöhen der Temperatur von -50°C auf 150°C bei einer Rate von 3°C/Minute unter Verwendung eines feuchtigkeitskonditionierenden Viskoelastometers erhalten wird, in einem Bereich von -10°C bis 15°C aufweist, und wobei die PVA-Folie weiterhin einen Plastifizierer (C) enthält, der Trimethylolpropan umfasst.

2. Folie nach Anspruch 1, die durch Bilden einer Folie aus einer Harzzusammensetzung [I] hergestellt wird, die zumindest zwei Arten von Polyvinylalkoholharzen (A) mit unterschiedlichen Hydrolysegraden enthält.

3. Folie nach Anspruch 2, wobei die Harzzusammensetzung [I] zwei Arten von Polyvinylalkoholharzen (A) mit unterschiedlichen Hydrolysegraden umfasst, und der Unterschied im Hydrolysegrad zwischen einem Polyvinylalkoholharz (a1) mit einem niedrigeren Hydrolysegrad und einem Polyvinylalkoholharz (a2) mit einem höheren Hydrolysegrad zumindest 3 Mol-% beträgt.

4. Folie nach Anspruch 3, wobei das Polyvinylalkoholharz (a1) einen Hydrolysegrad von nicht weniger als 70 Mol-% bis weniger als 82 Mol-% aufweist und das Polyvinylalkoholharz (a2) einen Hydrolysegrad von nicht weniger als 82 Mol-% aufweist.

5. Folie nach Anspruch 3 oder 4, wobei das Verhältnis von Polyvinylalkoholharz (a1) zu Polyvinylalkoholharz (a2) von 50/50 bis 90/10 Gew. -% beträgt.

6. Folie nach einem der Ansprüche 1 bis 5, die ferner einen anorganischen Füllstoff (B) mit einer durchschnittlichen Teilchengröße von 1 bis 10 µm enthält.

7. Verpackung einer Chemikalie, die eine Polyvinylalkoholfolie nach einem der Ansprüche 1 bis 6 und eine Chemikalie umfasst.

8. Verpackung nach Anspruch 7, wobei die beschickte Chemikalie bei normaler Temperatur eine Flüssigkeit ist.

## Revendications

1. Film en poly(alcool vinylique) (PVA) qui se dissout dans l'eau à 20 °C en l'espace de 10 minutes si un échantillon ayant une taille de 5 cm x 5 cm est découpé dans un film ayant une épaisseur comprise entre 5 et 100 µm et immergé dans 1 litre d'eau maintenue à 20 °C et agitée au moyen d'un agitateur, et qui a un rapport α/β non supérieur à 10, où α est le module de stockage du film à 20 °C dans une atmosphère sèche et β est le module de stockage du film à 20 °C sous 80 % HR, dans lequel le PVA est obtenu par polymérisation d'un composé ester vinylique choisi parmi le formiate de vinyle, l'acétate de vinyle, le trifluoroacétate de vinyle, le propionate de vinyle, le butyrate de vinyle, le caprate de vinyle, le laurate de vinyle, l'ester vinylique d'acide versatique, le palmitate de vinyle, et le stéarate de vinyle, en conséquence de quoi le film de PVA a une température de transition vitreuse, qui est la température maximale de dispersion principale obtenue lors d'une mesure en continu de la viscoélasticité dynamique à 20 °C et avec une teneur en humidité non supérieure à 1 000 ppm, à une fréquence de mesure de 2 Hz avec élévation de la température de -50 °C à 150 °C à une vitesse de 3 °C/minute par utilisation d'un viscoélastomètre à conditionnement de l'humidité, située dans la plage allant de -10 °C à 15 °C, et lequel film de PVA contient en outre un plastifiant (C) comprenant du triméthylolpropane.

2. Film selon la revendication 1, qui est préparé par formation d'un film à partir d'une composition de résine [I] contenant au moins deux types de résines de poly(alcool vinylique) (A) ayant des degrés d'hydrolyse différents.

3. Film selon la revendication 2, dans lequel ladite composition de résine [I] comprend deux types de résines de poly(alcool vinylique) (A) ayant des degrés d'hydrolyse différents, et la différence de degré d'hydrolyse entre une résine de poly(alcool vinylique) (a1) ayant un moindre degré d'hydrolyse et une résine de poly(alcool vinylique) (a2) ayant un degré d'hydrolyse plus élevé est d'au moins 3 % en moles.

4. Film selon la revendication 3, dans lequel ladite résine de poly(alcool vinylique) (a1) a un degré d'hydrolyse d'au moins 70 % en moles à moins de 82 % en moles, et ladite résine de poly(alcool vinylique) (a2) a un degré d'hydrolyse d'au moins 82 % en moles.

5. Film selon la revendication 3 ou 4, dans lequel le rapport de la résine de poly(alcool vinylique) (a1) à la résine de poly(alcool vinylique) (a2) est de 50/50 à 90/10 en poids.

6. Film selon l'une quelconque des revendications 1 à 5, qui contient en outre une charge inorganique (B) ayant une granulométrie moyenne de 1 à 10 µm.

7. Conditionnement d'un produit chimique comprenant un film en poly(alcool vinylique) selon l'une quelconque des revendications 1 à 6, et un produit chimique.

8. Conditionnement selon la revendication 7, dans lequel ledit produit chimique chargé est un liquide à la température ordinaire.
